# FASCICULE DE BREVET EUROPEEN

(11) **EP 0 624 082 B1**
(45) Date de publication et mention de la délivrance du brevet: **27.08.1997**
(21) Numéro de dépôt: 92904854.4
(22) Date de dépôt: 24.01.1992
(51) Int. Cl.: A61F 13/00, A61F 13/20

(54) **PRODUIT DE PANSEMENT A AME DE FIBRES EN ALGINATE**
VERBANDPRODUKT MIT KERN AUS ALGINAT-FASERN
DRESSING MATERIAL HAVING A FIBRE CORE FROM ALGINATE - FIBRES

(43) Date de publication de la demande: 17.11.1994
(73) Titulaire: Société PRECIS, 92000 Nanterre Hauts-de-Seine (FR)
(72) Inventeur: BARIKOSKY, Michel, F-75007 Paris (FR)
(74) Mandataire: Bloch, Gérard
(86) Numéro de dépôt international: FR9200063
(87) Numéro de publication internationale: WO9314724

(56) Documents cités:
- EP-A- 0 249 937
- EP-A- 0 344 913
- GB-A- 495 461
- GB-A- 855 537
- US-A- 4 705 514

## Description

La présente invention concerne un produit de pansement, ou pansement, pour plaie à liquide biologique, comprenant sous une forme sensiblement cylindrique, une âme de fibres d'un alginate de métal.

Les pansements de ce type, généralement en forme de mèche, sont souvent utilisés pour des plaies humides, notamment profondes, telles que des cavités qui exsudent comme les escarres ou autres plaies post-opératoires.

Comme alginate de métal, on considère fréquemment un alginate d'un métal choisi dans la famille des métaux multivalents, à l'exception du magnésium et plus particulièrement l'alginate de calcium.

Une plaie provoque une perte de substance, ou de liquide biologique (sang ou exsudat). Appliqué dans une plaie, le pansement commence par absorber le liquide biologique qui suinte, ou qui exsude, les molécules d'eau du liquide s'intercalant entre les macromolécules de l'alginate. Une fois gonflé par absorption, le pansement est soumis à une gélification par échange ionique. Dans le cas de fibres d'alginate de calcium, elles cèdent des ions Ca²⁺ au liquide biologique qui leur cède des ions Na⁺. Au fur et à mesure que s'établit l'équilibre entre le calcium et le sodium, les fibres d'alginate perdent en partie leur structure cristalline. La gélification du pansement entraîne l'assèchement de la plaie et évite qu'il n'adhère aux tissus sous-jacents. Mais, la gélification provoque aussi un délitement et plus généralement une altération de l'intégrité de la structure mécanique de l'âme préjudiciable à son retrait de la plaie dans des conditions indolores.

On connaît, par le document EP-A-0 344 913, des pansements en alginate sous forme de mèche. Mais une telle mèche manque de cohésion, car l'alginate est biodégradable par les fluides biologiques.

On connaît aussi, par le document EP-A-0 249 937, une tige absorbante pour chirurgie dentaire en divers filaments possibles, notamment en filaments d'acétate, enveloppés d'un ruban perméable à l'eau. Mais ce n'est pas un produit de pansement.

La présente invention vise à proposer un produit de pansement du type mentionné ci-dessus, à pouvoir d'absorption, de retention et de gélification intact mais qui puisse facilement être retiré d'une plaie et de façon indolore.

A cet effet, le produit de pansement de l'invention est caractérisé par le fait que l'âme est entourée d'une enveloppe de maintien de structure perméable au liquide biologique et biocompatible avec la plaie.

Ainsi, et tout en maintenant la cohésion mécanique de l'âme fibrillaire, l'enveloppe n'altère nullement l'action d'absorption, de dilatation et de gélification visant à extraire le liquide biologique, ni l'action de rétention de protéines et autres déchets cellulaires et bactériens qui, sinon, entraveraient le phénomène de cicatrisation.

L'enveloppe peut être filamenteuse, à un fil ou à plusieurs. A un fil, il peut lier les fibres de l'âme par maillage, notamment par des points dits de chaînette, par enroulage, en particulier avec des spires jointives par exemple. A plusieurs fils, ils peuvent être maillés, tressés, torsadés, guipés autour de l'âme, par exemple.

L'enveloppe peut aussi être une gaine poreuse ou une gaine ajourée.

Comme matériaux d'enveloppement biocompatibles, on peut considérer, par exemple les polymères biocompatibles comme les polyesters, les polyamides, la viscose, ainsi d'ailleurs que les polymères biorésorbables.

Comme alginate de métal, pour l'âme, on peut considérer un alginate d'un métal choisi dans la famille des métaux multivalents, à l'exception du magnésium, et surtout, l'alginate de calcium.

L'invention sera mieux comprise à l'aide de la description suivante de plusieurs formes de réalisation du produit de pansement de l'invention, en référence au dessin annexé, sur lequel
- la figure 1 représente, en perspective une mèche avec deux fils tressés de maintien ;
- la figure 2 représente, de profil, une mèche avec un fil de maintien enroulé à spires jointives ;
- la figure 3 représente, de profil, une mèche avec un fil de maintien maillé par points de chaînette ;
- la figure 4 représente, de profil, une mèche avec une gaine de maintien ajourée et
- la figure 5 représente, de profil, une mèche avec une gaine de maintien poreuse.

La technique de réalisation d'une âme, ou mèche, de fibres d'alginate de métal, et plus particulièrement d'alginate de calcium, pour plaie à liquide biologique, est maintenant bien éprouvée et elle ne sera donc pas abordée ici, pas plus que la structure, texturée ou non, d'une telle mèche. Qu'il soit seulement précisé qu'une mèche se présente sous une forme grossièrement cylindrique et allongée. Pour éviter qu'elle ne se délite, qu'elle ne se désagrège, bref, qu'elle ne perde sa cohésion mécanique, il est prévu, dans les formes de réalisation représentées sur le dessin, qu'elle soit maintenue par une enveloppe perméable au liquide biologique et biocompatible avec la plaie à panser.

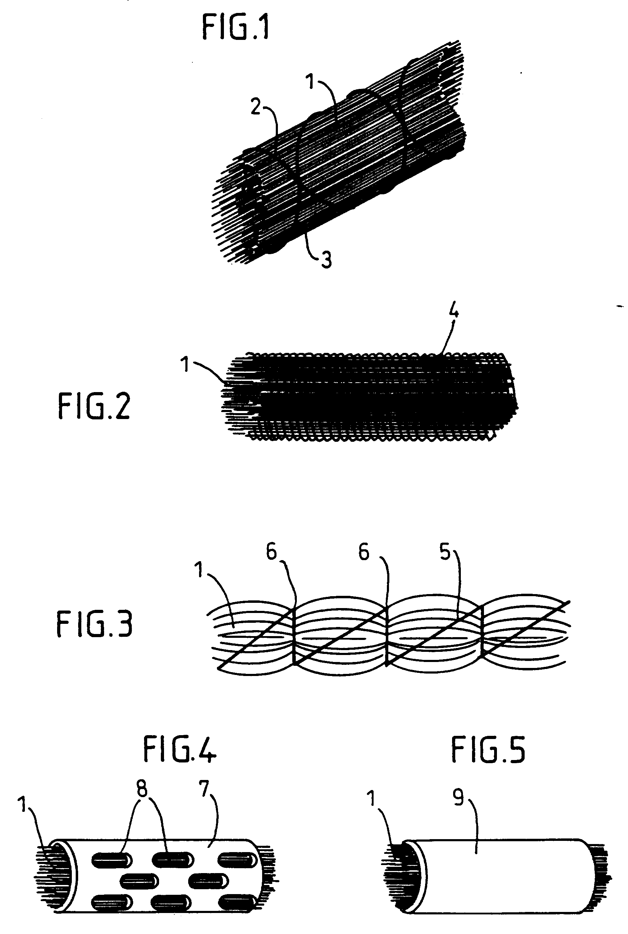

Dans la forme de la figure 1, la mèche 1 est maintenue par deux fils 2, 3 tressés autour de la mèche selon un pas relativement grand.

Dans la forme de la figure 2, la mèche 1 est maintenue par un seul fil 4 enroulé autour d'elle à spires presque jointives.

Dans la forme de la figure 3, la mèche 1 est maintenue par un seul fil 5 maillé par points de chaînette 6 régulièrement espacés.

Dans la forme de la figure 4, la mèche 1 s'étend à l'intérieur d'une gaine-enveloppe 7 percée de lumières 8 de passage (drainage) du liquide biologique.

Dans la forme de la figure 5, la mèche 1 s'étend à l'intérieur d'une gaine-enveloppe 9 à paroi poreuse pour le passage du liquide biologique.

Dans toutes les formes de réalisation décrites ci-dessus le liquide biologique reste parfaitement drainé par la mèche fibrillaire. Les fils ou les gaines qui l'enveloppent évitent sa delitescence ainsi que son contact intime avec la plaie. Les substances piégées par le gel fibrillaire ne sont donc pas davantage au contact de la plaie. La mèche peut ensuite être facilement retirée de la plaie de façon indolore.

## Revendications

1. Produit de pansement pour plaie à liquide biologique, comprenant, sous une forme sensiblement cylindrique, une âme (1) de fibres d'alginate de métal, caractérisé par le fait que l'âme (1) est entourée d'une enveloppe (2,3;4;5;7;9) de maintien de structure perméable au liquide biologique et biocompatible avec la plaie.

2. Produit selon la revendication 1, dans lequel l'enveloppe (2,3;4;5) est filamenteuse.

3. Produit selon la revendication 2, dans lequel l'enveloppe comporte un seul fil (4).

4. Produit selon la revendication 2, dans lequel l'enveloppe comporte plusieurs fils (2,3).

5. Produit selon la revendication 1, dans lequel l'enveloppe est constituee d'une gaine (7;9).

6. Produit selon la revendication 5, dans lequel la gaine (7) est ajourée.

7. Produit selon la revendication 5, dans lequel la gaine (9) est poreuse.

8. Produit selon l'une des revendications 1 à 7, dans lequel l'enveloppe est en polymère.

9. Produit selon l'une des revendications 1 à 8, dans lequel les fibres de l'âme (1) sont en alginate d'un metal choisi dans la famille des métaux multivalents, à l'exception du magnésium.

10. Produit selon la revendication 9, dans lequel les fibres sont en alginate de calcium.

## Claims

1. Dressing product for a wound containing a biological liquid, comprising, in a substantially cylindrical form, a core (1) of fibres of metal alginate, characterised by the fact that the core (1) is surrounded by a retaining wrapper (2, 3; 4; 5; 7; 9) whereof the structure is permeable to the biological liquid and biocompatible with the wound.

2. Product according to Claim 1, in which the wrapper (2, 3; 4; 5) is made up of filaments.

3. Product according to Claim 2, in which the wrapper comprises a single thread (4).

4. Product according to Claim 2, in which the wrapper comprises several threads (2, 3).

5. Product according to Claim 1, in which the wrapper is constituted by a sheath (7; 9).

6. Product according to Claim 5, in which the sheath (7) is perforated.

7. Product according to Claim 5, in which the sheath (9) is porous.

8. Product according to one of Claims 1 to 7, in which the wrapper is made of polymer.

9. Product according to one of Claims 1 to 8, in which the fibres of the core (1) consist of an alginate of a metal chosen from the family of multivalent metals, with the exception of magnesium.

10. Product according to Claim 9, in which the fibres consist of calcium alginate.

## Patentansprüche

1. Verbandserzeugnis für eine Wunde mit biologischer Flüssigkeit, das in einer im wesentlichen zylindrischen Gestalt einen Kern (1) aus Metallalginatfasern aufweist,
dadurch gekennzeichnet,
daß der Kern (1) von einer Umhüllung (2; 3; 4; 5; 7; 9) zur Halterung einer Struktur umgeben ist, die für biologische Flüssigkeiten durchlässig und mit der Wunde biokompatibel ist.

2. Erzeugnis nach Anspruch 1,
wobei die Umhüllung (2; 3; 4; 5) faserig ist.

3. Erzeugnis nach Anspruch 2,
wobei die Umhüllung einen einzigen Faden (4) aufweist.

4. Erzeugnis nach Anspruch 2,
wobei die Umhüllung mehrere Fäden (2; 3) aufweist.

5. Erzeugnis nach Anspruch 1,
wobei die Umhüllung als Hülle (7; 9) ausgebildet ist.

6. Erzeugnis nach Anspruch 5,
wobei die Hülle (7) durchbrochen bzw. mit Öffnungen versehen ist.

7. Erzeugnis nach Anspruch 5,
wobei die Hülle (9) porös ist.

8. Erzeugnis nach einem der Ansprüche 1 bis 7,
wobei die Umhüllung aus einem Polymer besteht.

9. Erzeugnis nach einem der Ansprüche 1 bis 8,
wobei die Fasern des Kerns (1) aus einem Alginat eines Metalles bestehen, das aus der Familie der mehrwertigen Metalle, mit Ausnahme von Magnesium, ausgewählt ist.

10. Erzeugnis nach Anspruch 9,
wobei die Fasern aus Calciumalginat betehen.
